# EUROPÄISCHE PATENTSCHRIFT

(11) **EP 0 749 336 B1**
(45) Veröffentlichungstag und Bekanntmachung des Hinweises auf die Patenterteilung: **10.06.1998**
(21) Anmeldenummer: 95910530.5
(22) Anmeldetag: 27.02.1995
(51) Int. Cl.: A61M 37/00

(54) **KANÜLE ZUM SUBKUTANEN ABLEGEN EINES ELEMENTES**
CANNULA FOR THE SUBCUTANEOUS DEPOSITION OF AN OBJECT
CANULE D'IMPLANTATION SOUS-CUTANEE D'UN ELEMENT

(30) Priorität: 25.02.1994 DE 9403161 U
(43) Veröffentlichungstag der Anmeldung: 27.12.1996
(73) Patentinhaber: SÜDDEUTSCHE FEINMECHANIK GMBH, D-63607 Wächtersbach (DE)
(72) Erfinder: SCHLEGEL, Karl-Heinz, D-63505 Langenselbold (DE); FEIT, Herbert, D-63607 Wächtersbach (DE)
(74) Vertreter: Stoffregen, Hans-Herbert, Dr. Dipl.-Phys.
(86) Internationale Anmeldenummer: EP9500713
(87) Internationale Veröffentlichungsnummer: WO9523010

(56) Entgegenhaltungen:
- WO-A-88/06905
- WO-A-92/15362

## Beschreibung

Die Erfindung bezieht sich auf eine Kanüle zum Ablegen eines Elementes in einem Körper eines Lebewesens, umfassend einen eine erste Handhabe aufweisenden Kanülenhalter und eine diesen zumindest bereichsweise umgebende und zu diesem axial verschiebbare, einen sich in der Kanüle erstreckenden Mandrin aufweisende Hülse mit zweiter Handhabe, wobei das Element wie medizinisches Langzeitpräparat, Identifikationsträger oder eine radioaktive Substanz enthaltende Kapsel durch Relativbewegung zwischen dem Mandrin und der das Element aufnehmenden Kanüle ablegbar ist.

Eine entsprechende Kanüle ist der WO 92/15362 zu entnehmen. Mit einer entsprechenden Kanüle ist es erstmals möglich gewesen, das Element vor dem Ablegen in einer vorher bekannten Position anzuordnen, um so das Implantieren zu vereinfachen und das Ablegen in einem gewünschten Gewebe sicherzustellen. Dabei erfolgte die Lagefixierung insbesondere durch einen kanülenspitzenseitig von einem die Kanüle zumindest bereichsweise verschließenden Verschluß in Form eines einen medizinischen Wirkstoff enthaltenden salbenartigen Materials, eines Klebstoffmaterials oder eines Silikonpfropfens.

Durch die Art der Lagefixierung bedingt werden beim Implantieren des Elements Fremdstoffe in den Körper eingebracht, die gegebenenfalls zu nicht gewünschten oder unerwarteten Reaktionen führen können.

Weitere Kanülen zum Ablegen von festen oder halbfesten Präparaten unter die Haut eines Lebewesens sind zum Beispiel der US 4,900,304, US 4,950,234 oder der EP 0 255 123 A2 zu entnehmen.

In der EP 0 304 107 A1 wird eine Injektionskanüle zum Ablegen eines Implantats beschrieben, wobei die Kanüle selbst zum einmaligen Gebrauch bestimmt ist.

Allein zu Transportzwecken ist nach der US 4, 820,267 ein Implantat in einer Kanüle lagefixiert, und zwar von einem Mandrin und distal von einem Stift. Damit der Stift nicht unkontrolliert aus der Kanüle herausrutschen kann, wird dieser außenseitig von einer Schutzhülle festgelegt.

Zur Identifizierung von Tieren ist nach der WO 90/05488 eine flexible Kanüle vorgesehen; deren distaler Bereich einen geringeren Querschnitt als derjenige aufweist, in dem ein Mandrin verschiebbar ist.

Aus der US 4,846,793 oder der US 4,994,028 ist eine Kanüle der eingangs beschriebenen Art bekannt, bei der die Hülse zum Kanülenhalter verdrehbar ausgebildet ist, um eine gegenseitige Verrastung vorzunehmen. Hierdurch ergibt sich der Nachteil, daß das Einstechen der Kanüle und anschließende Ablegen des Elements grundsätzlich nur mit zwei Händen erfolgen kann.

Aufgabe der vorliegenden Erfindung ist es, eine Kanüle der eingangs genannten Art so weiterzubilden, daß einerseits eine problemlose und einwandfreie Lagefixierung des zu implantierenden Elementes möglich ist, ohne daß Fremdsubstanzen erforderlich sind, die beim Implantieren mit in den Körper eindringen können, in dem das Element implantiert wird. Auch soll die Möglichkeit gegeben werden, auf einfache Weise die Lage des Elementes zu überprüfen, auch dann, wenn die Kanüle aus nichtdurchscheinendem Material und insbesondere aus Metall besteht.
Andererseits soll die Möglichkeit bestehen, das Einstechen der Kanüle in den Körper und das anschließende Ablegen des Elements durch Zurückziehen der Kanüle mit einer Hand durchführen zu können.

Bezüglich der Möglichkeit einer Einhandbedienung wird die Aufgabe dadurch gelöst, daß bei Druck auf die zweite von der Hülse ausgehende Handhabe die Hülse mit dem Kanülenhalter durch zumindest ein von diesem ausgehendes Spreizelement oder ein von dem Kanülenhalter oder der Hülse ausgehendes Rast- oder Hebelelement verrastbar ist.

Durch diese Maßnahme ist sichergestellt, daß beim Einstechen der Kanüle in den Körper diese zu der Hülse und damit dem Mandrin dann nicht oder nur unwesentlich verrücken kann, wenn der zum Einstechen erforderliche Druck von der Hülse, also der zweiten Handhabe wie Griffstück ausgeht.

Um das Element abzulegen, bedarf es sodann nur eines axialen Verschiebens des Kanülenhalters in Richtung der zweiten Handhabe, wodurch entweder automatisch das von dem Kanülenhalter ausgehende Rastelement in Ausgriff zur Hülse gelangt oder aber das Hebelelement mit der Hand, die den Kanülenhalter axial in Richtung der zweiten Handhabe zieht, kontrolliert in Ausgriff mit der Hülse gebracht wird.

Insbesondere ist vorgesehen, daß der Kanülenhalter einen unverrückbaren, die Kanüle umgebenden Ansatz und einen diesen umgebenden und relativ zu diesem axial verschiebbaren die erste Handhabe aufweisenden Hülsenkörper umfaßt, daß von dem Ansatz zumindest ein den Hülsenkörper durchsetzendes, bei Druck auf die zweite Handhabe gegen einen Abschnitt der Hülse sich abstützendes Spreizelement ausgeht und daß bei Bewegung der ersten Handhabe in Richtung der zweiten Handhabe der Hülsenkörper das Spreizelement in Ausgriff mit dem Abschnitt bringt und den Ansatz mitzieht.

Alternativ besteht die Möglichkeit, daß von der Hülse ein verschwenkbares Rastelement wie Hebel ausgeht, das gegen den Kanülenhalter bzw. dessen Handhabe abstützbar ist.

Dabei ist das Rastelement vorzugsweise ein Winkelhebel, dessen kurzer Schenkel im Bereich der zweiten Handhabe, vorzugsweise entlang dessen äußerer der Kanüle abgewandten Fläche verläuft und dessen langer Schenkel mit seinem freien Ende auf der Kanüle abgewandter Seite der ersten Handhabe abstützbar ist.

Dabei kann der Winkelhebel in einer von der zweiten Handhabe ausgehenden und Schwenkachse für den Winkelhebel bildenden Aufnahme festklemmbar sein.

Um ein einfaches Verschwenken des Winkelhebels zu ermöglichen, sollte der kurze Schenkel mit seinem freien Endbereich an der zweiten Handhabe abgestützt sein und zwischen der Aufnahme und der Abstützung beabstandet zur zweiten Handhabe verlaufen. Um den langen Schenkel in Ausgriff mit dem Kanülenhalter bzw. dessen Griffstück zu bringen, ist es dann nur noch erforderlich, daß auf den kurzen Schenkel ein Druck ausgeübt wird, wodurch automatisch der lange Schenkel von der Kanüle wegbewegt wird. Der kurze Schenkel kann dabei mit einem Abschnitt, vorzugsweise mit seinem freien Ende an der zweiten Handhabe festgerastet werden, wodurch sichergestellt ist, daß ein unkontrolliertes Zurückschwenken des Winkelhebels nicht erfolgen kann.

Ein weiterer selbständiger Lösungsvorschlag sieht vor, daß von dem Kanülenhalter ein außenseitig entlang der Hülse verlaufender und mit dieser verrastbares Hebelelement ausgeht. Dabei kann das Hebelelement vorzugsweise über ein Scharnier wie Filmscharnier mit der ersten Handhabe verbunden sein. Zum Verschwenken - also Inausgriffbringen mit der Hülse - geht von dem Hebelelement eine Handhabe wie Vorsprung aus, die bzw. der dann erfaßt wird, wenn das Element nicht mehr in Eingriff mit der Hülse sein soll. Vorzugsweise erstreckt sich das Hebelelement tangential in der Hülse - auch ein radialer Verlauf ist möglich -, wobei die Handhabe in diesem Bereich des Hebelelements verläuft und über der Hülse vorsteht.

Das Hebelelement selbst kann mit einem endseitigen Vorsprung in der Hülse arretierbar sein, wobei vorzugsweise bei zu der Hülse arretierter Kanülenhalterung der endseitige Vorsprung die Hülse durchsetzt und in eine Aussparung des Kanülenhalters eingreift.

Ist das Hebelelement vorzugsweise in Richtung der Hülse vorzugsweise werkstoffbedingt federvorgespannt, wodurch ein automatisches Verrasten zwischen dem Kanülenhalter und der Hülse beim Einstechen der Kanüle erfolgt, so besteht alternativ die Möglichkeit, daß das Hebelelement bei fehlender Krafteinwirkung auf dieser beabstandet zur Hülse verläuft, so daß das Verrasten kontrolliert erfolgen muß.

Losgelöst von der Ausbildung der Verrastung ist jedoch stets die Möglichkeit gegeben, daß mit ein und derselben Hand sowohl das Einstechen durch Druckeinwirkung auf die zweite Handhabe, Lösen der Verrastung bzw. Arretierung und sodann das Zurückziehen der Kanüle durch Zurückziehen des Griffstücks in deren axialer Richtung erfolgen kann. Das Lösen kann automatisch zum einen bei Verwendung von Spreizelementen oder zum anderen durch derartiges Festlegen des Hebelelementes in der Hülse bzw. dem Kanülenhalter dann erfolgen, wenn bei Druckeinwirkung auf die zweite Handhabe das Hebelelement verrastet bleibt, jedoch beim Ziehen der ersten Handhabe in Richtung der zweiten Handhabe sich aus der Arretierung löst. Selbstverständlich kann ein Endarretieren auch durch kontrolliertes Verschieben bzw. Verstellen des Hebelelementes erfolgen.

Um den Teilaspekt der Erfindung, der sich auf die problemlose und einwandfreie Lagefixierung des zu implantierenden Elements bezieht, zu lösen, wird vorgeschlagen, daß das zumindest außenseitig nachgiebige Eigenschaften aufweisende Element von einem Abschnitt der Kanüle festgeklemmt ist, in dem die Kanüle im Vergleich von zwischen dem Element und der Kanülenspitze vorhandenem Querschnitt einen abweichenden Querschnitt oder eine Durchbrechung aufweist. Dabei kann die Querschnittsverringerung durch eine zumindest bereichsweise umlaufende Sicke gebildet werden. Aber auch andere Möglichkeiten zur eine Querschnittsveränderung durch Körnung eines Kanülenwandabschnitts oder durch Ausbilden einer Durchbrechung sind möglich. Ein Stauchen der Kanüle zur Erzielung der Querschnittsänderung, durch die das zu implantierende Element festgeklemmt wird, ist ebenfalls denkbar.

Ferner kann im Bereich des das Element festklemmenden Abschnitts der Kanüle eine Durchbrechung als Sichtfenster ausgebildet sein, durch die selbst die Querschnittsänderung oder ein Raum vorgegeben wird, in dem sich das Element ausdehnen kann, wodurch ebenfalls eine Lagefixierung erfolgt.

Durch die erfindungsgemäße Lehre erfolgte auf einfachem Wege eine Lagefixierung des Elementes, indem die Kanüle in dem Bereich, in dem das Element vor seiner Implantation lagefixiert werden soll, eine Querschnittsänderung erfährt. Da das zu implantierende Element zumindest außenseitig nachgiebige bzw. elastische Eigenschaften aufweist, ist ein sicheres Festklemmen möglich, ohne daß die Gefahr einer Beschädigung des Elementes erfolgt, insbesondere dann, wenn von der Außenhülle medizinische oder radioaktive Präparate eingeschlossen sind.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung eines der Zeichnung zu entnehmenden bevorzugten Ausführungsbeispiels.

Es zeigen:
- Fig. 1: einen Schnitt durch eine Kanüle zum Implantieren eines Elementes wie medizinischen Präparats oder Identifikationsträgers,
- Fig. 2: einen Ausschnitt aus der Kanüle nach Fig. 1, jedoch in vergrößerter Darstellung,
- Fig. 3: einen weiteren Ausschnitt der Kanüle nach Fig 1,
- Fig. 4: einen Längsschnitt durch eine weitere Ausführungsform einer Kanüle,
- Fig. 5: einen Ausschnitt der Kanüle nach Fig. 4, jedoch um 90° gedreht,
- Fig. 6: einen Schnitt entlang der Linie AB in Fig. 5,
- Fig. 7: eine dritte Ausführungsform einer Kanüle,
- Fig. 8: einen Schnitt entlang der Linie in Richtung der Linie CD in Fig. 7 und
- Fig. 9: eine alternative Lösung einer Arretierung zu dem der Fig. 7 zu entnehmenden Vorschlag.

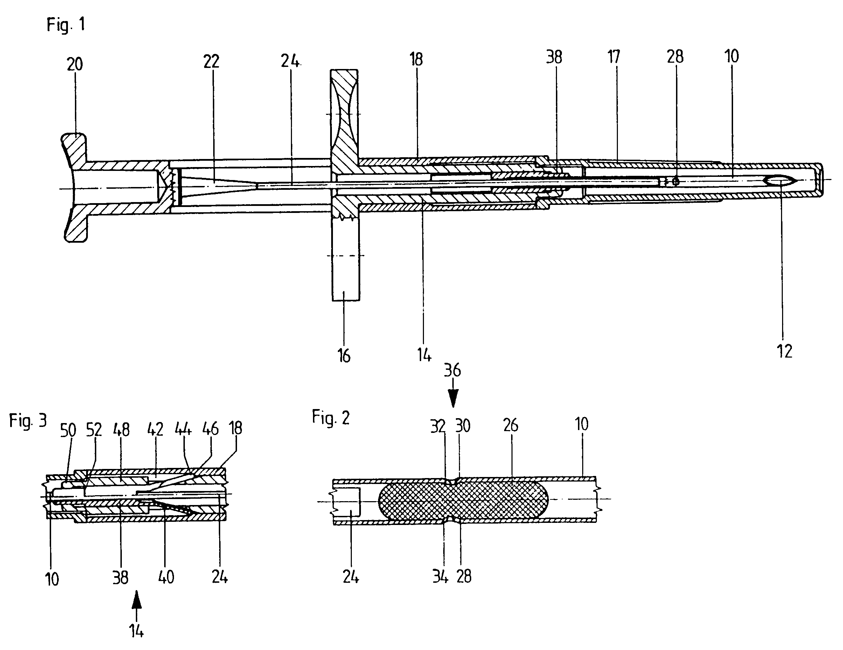

Um in einem tierischen oder menschlichen Lebewesen ein Element wie medizinisches Präparat, insbesondere Langzeitpräparat wie Reptid, ein eingekapseltes radioaktives Präparat (Slak) oder einen Identitätsträger zu implantieren, wird eine Kanüle (10) benutzt. Bei der Kanüle (10) kann es sich um eine Stahlkanüle, eine Kunststoffkanüle oder um eine Kanüle anderen geeigneten Materials mit vorzugsweise schräg angeschliffener Spitze (12) handeln. Die Kanüle (10) wird von einem Kanülenhalter (14) aufgenommen, der im Ausführungsbeispiel in eine Handhabe wie Griffstücke (16) übergeht. Vor und hinter den Griffstücken (16) erstreckt sich eine Hülse (18), die kanülenabgewandt geschlitzt ist, um einen in Längsrichtung der Kanüle (10) zu verschiebenden und außenseitig gleichfalls mit einer Handhabe (20) versehenen Auswurfkolben (22) zu bewegen, der seinerseits mit einem zu der Kanüle (10) zu verschiebenden Mandrin (24) verbunden ist, über den das in Fig. 1 nicht dargestellt und in Fig. 2 vergrößert wiedergegebene Element (26) durch Relativverschiebung zwischen Mandrin und Kanüle (10) in einem Körper abgelegt werden kann. Wird vorzugsweise das Element (26) durch Zurückziehen der Kanüle (10) abgelegt, so kann selbstverständlich auch ein Ablegen durch Verschiebung des Mandrin (24) in Richtung der Kanülenspitze (12) erfolgen.

Die Kanüle (10) ist außerdem mit einer Schutzkappe (17) umgeben, die auf den Kanülenhalter (14) aufgesteckt ist.

Zwischen dem Griffstück (16) und der Kanülenspitze (12) weist die Kanüle (10) eine Geometrieveränderung wie Lumeneinschnürung (28) auf, die auf unterschiedliche Weise hergestellt werden kann. So kann die Kanülenwandung gekörnt oder mit einer zumindest bereichsweise umlaufenden Sicke versehen sein. Insbesondere ist vorgesehen, daß durch zum Beispiel Körnungen zwei diametral zueinander verlaufende Durchbrechungen (28) und (30) hergestellt werden, deren Innenränder (32) bzw. (34) sich in den Innenraum der Kanüle (10) erstrecken, also die gewünschten Geometrieveränderungen oder Querschnittsverringerungen bewirken.

In dem die Querschnittsänderungen aufweisenden Abschnitt (36) der Kanüle (10) wird nun das zu implantierende Element (26) festgeklemmt. Hierzu ist es erforderlich, daß das Element zumindest außenseitig nachgiebige bzw. elastische Eigenschaften aufweist, um sicherzustellen, daß das Element selbst nicht beschädigt wird, also zum Beispiel von einer Hülle aufgenommene Präparate oder radioaktive Substanzen nicht unkontrolliert entweichen können.

Wie anhand der Fig. 1 und 2 des weiteren verdeutlicht werden soll, bieten die Durchbrechungen (28) und (30) gleichzeitig die Möglichkeit einer optischen Überprüfung des festgeklemmten Elementes (26), haben also die Funktion von Sichtfenstern. In die Durchbrechungen (28), (30) kann sich außerdem das Element hineindrücken, so daß eine Lagefixierung erfolgt. Zusätzliche Lumenänderungen sind in diesem Fall nicht erforderlich.

Um sowohl das Einstechen der Kanüle als auch das Ablegen des Elementes (26) mit nur einer Hand vornehmen zu können, besteht erfindungsgemäß die Möglichkeit, daß während des Einstechens die Hülse (18) zu der Kanüle (10) und damit der Kanülenhalterung (14) verrastbar ist, wohingegen beim anschließenden Zurückziehen der Kanüle (10), also des Bewegens des Griffstücks (16) in Richtung der Handhabe (20) allein eine axiale Bewegung erforderlich ist.

Um dieses Verrasten zu ermöglichen, sind verschiedene Lösungsmöglichkeiten vorgesehen.

So kann der Kanülenansatz (38) Spreizelemente (40) aufweisen, deren freie Enden (44) sich in Richtung des Griffstücks (16) erstrecken und in Richtung der Hülse (18) federnd abragend sind, wobei bei Druckeinwirkung auf die Handhabe (20) das freie Ende (44) des Spreizelementes (40) an einen, Abschnitt wie Absatz (46) der Innenwandung der Hülse (18) anliegt.

Hierzu gehen die Spreizelemente (40) von einem die Kanüle (10) unverrückbar umgebenden Kanülenansatz (38) aus, der von einem Hülsenkörper (48) umgeben ist, der das Griffstück (16) aufweist.

Der Kanülenansatz (38) und der Hülsenkörper (48) bilden den Kanülenhalter (14), wobei das Spreizelement (40) eine Durchrechung (42) des Hülsenkörpers (48) dann durchsetzen kann, wenn das freie Ende (44) des Spreizelements (40) an dem Absatz (46) der Hülse (18) anliegen soll.

Soll das Element (26) abgelegt werden, so wird erwähntermaßen das Griffstück (16) axial zurückgezogen, wodurch sich der Hülsenkörper (48) zunächst entlang des Kanülenansatzes (38) bewegt, bevor ein ringförmiger Vorsprung (50) des Hülsenkörpers (48) an einer Stufe (52) des Kanülenansatzes (38) anliegt und diesen bei weiterer axialer Bewegung des Kanülenhalters (14) mitzieht.

Durch die zunächst erfolgende Relativbewegung zwischen dem Hülsenkörper (48) und dem Kanülenansatz (38) in Richtung des freien Endes (44) des Spreizelementes (40) wird dieses zur Kanüle (10) hin gebogen, so daß das freie Ende (44) in Ausgriff mit dem Absatz (46) der Hülse (18) gelangt. Somit kann der Kanülenhalter (14) gegenüber der Hülse (18) axial verschoben werden.

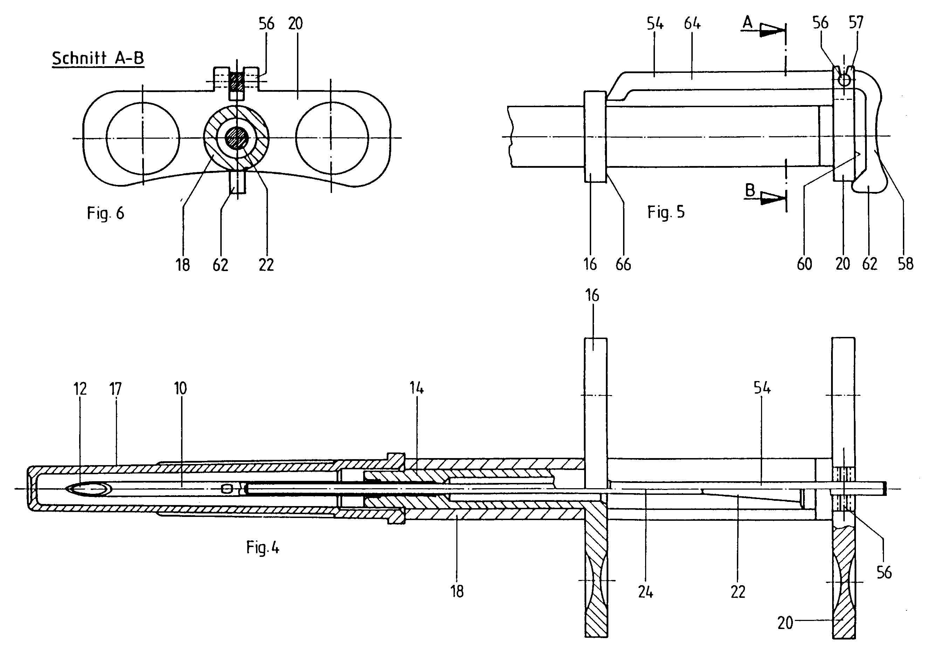

Andere Möglichkeiten einer Arretierung zwischen der Hülse (18) und dem Kanülenhalter (14) ergeben sich aus den Fig. 4 bis 9. Dabei ergibt sich der Vorteil, daß der Kanülenhalter (14) mit seinem innerhalb der Hülse (18) verschiebbaren Abschnitt nicht mehrteilig ausgebildet sein muß, also keine relativ zueinander verschiebbaren Abschnitte umfaßt wie dies bei der Ausführungsform der Fig. 1 und 2 der Fall ist.

Bei dem Ausführungsbeispiel der Fig. 4 bis 6 geht von dem Griffstück (20) der den Auswurfkolben (22) aufweisenden Hülse (18) ein Winkelhebel (54) aus, der auf dem Griffstück (16) des Kanülenhalters (14) abstützbar ist, so daß bei Druckeinwirkung auf das Griffstück (20) in Richtung der Kanülenspitze (12) die Hülse (18) mit dem Kanülenhalter (14) als Einheit axial verschiebbar sind.

Der Winkelhebel (54) ist um eine Achse (56) schwenkbar, die von einer Aufnahme (57) gebildet wird, in die der Winkelhebel (54) einrastbar ist. Die Aufnahme (57) geht von dem Griffstück (20) der Hülse (18) aus.

Der kurze Schenkel (58) des Winkelhebels (54) erstreckt sich entlang der der Kanülenspitze (12) abgewandten Fläche (60) des Griffstücks (20) und verläuft zu diesem bereichsweise beabstandet. Der kurze Schenkel (58) weist einen vorzugsweise wulstartigen Endabschnitt (62) auf, der dann in dem Griffstück (20) einrasten kann, wenn der Winkelhebel (54) in nachstehend beschriebener Art entarretiert werden soll. Alternativ besteht die Möglichkeit; den Winkelhebel (54) über zum Beispiel seinen kurzen Schenkel (58) derart zu arretieren, daß ein unkontrolliertes Verschwenken des Winkelhebels (54) ausgeschlossen ist.

Selbstverständlich ist es nicht zwingend erforderlich, daß der kurze Schenkel (58) irgendwann arretiert ist. Der Schenkel sollte jedoch zunächst am Griffstück (20) abgestützt sein.

Sofern auf den kurzen Schenkel (58) des Winkelhebels (54) kein Druck ausgeübt wird, liegt der Winkelhebel (54) mit seinem langen Schenkel (64) an der Außenseite (66) des Griffstücks (16) an, wie die Fig. 5 verdeutlicht, bzw. ist mit diesem arretiert. Somit kann das Einstechen der Kanüle (10) in ein Gewebe erfolgen, ohne daß sich die Kanüle (10) zu dem Griffstück (20) und damit dem Mandrin (24) verschiebt. Soll das sich in der Kanüle (10) befindliche Element (26) abgelegt werden, wird auf den kurzen Schenkel (58) des Winkelhebels (54) ein Druck ausgeübt bzw. dieser wird entarretiert, so daß infolgedessen der lange Schenkel (64) um die Achse (56) nach außen verschwenkt wird und somit in Ausgriff mit dem Griffstück (16) gelangt. Sodann kann durch axiales Zurückziehen des Griffstücks (16) das Element (26) abgelegt werden, da der Mandrin (24), der das Element (26) festhält, verharrt und die Kanüle (10) gegenüber diesem zurückgezogen wird.

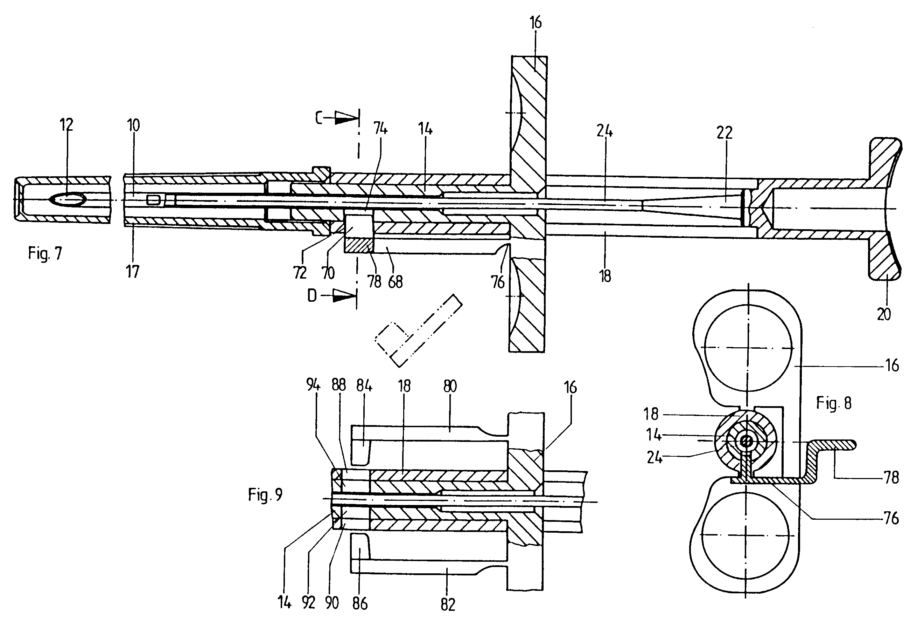

Die Arretierung nach dem Ausführungsbeispiel der Fig. 7 bis 9 erfolgt über einen Hebel (68), der von dem Griffstück (16) der Kanülenhalterung (14) ausgeht und mit einem Endabschnitt (70) in Form eines sich in Richtung der Kanüle (10) erstreckenden Vorsprungs in eine angepaßte Aussparung (72) der Hülse (18) tangential oder radial eingreifen kann. Dann kann der Abschnitt (70) vorzugsweise die Hülse (18) vollständig durchsetzen und in eine Aussparung (74) des Kanülenhalters (14) eingreifen, um die Arretierung zu verstärken, insbesondere dann, wenn die Wandung der Hülse (18) dünn ist.

Das Hebelelement (68) ist vorzugsweise über eine Art Filmscharnier (76) mit dem Griffstück (16) verbunden und in Richtung der Hülse werkstoffbedingt federvorgespannt. Hierdurch zeigt der Vorsprung (70) das Bestreben, stets an der Hülse (18) anzuliegen.

Um ein Entarretieren zu ermöglichen, also den Hebel (68) aus den Aussparungen (72) und (74) zu entfernen, geht von dem Hebel (68) bzw. dem Vorsprung eine Handhabe (78) aus, die seitlich entlang der Hülse (18) verläuft und/oder radial absteht, so daß kein Erfassen der Handhabe wie Vorsprung (78) das Hebelelement (68) in Ausgriff mit der Hülse (18) gebracht wird.

Alternativ zu dem Ausführungsbeispiel der Fig. 7 können von den, Griffstück (16) diametral zueinander verlaufende Hebelelemente (80), (82) ausgehen, die jedoch nicht in Richtung der Hülse (18) vorgespannt sind. Vielmehr verlaufen diese mit ihrer in Richtung der Hülse (18) sich erstreckenden Endabschnitten (84), (86) beabstandet zur Außenfläche der Hülse. Soll ein Arretieren erfolgen, muß auf die Hebel (80), (82) in Richtung auf die Hülse (18) ein Druck ausgeübt werden, damit die Vorsprünge (84), (86) in entsprechende Aussparungen (88), (90) bzw. (92), (94) der Hülse (18) bzw. des Kanülenhalters (14) eingreifen.

## Patentansprüche

1. Kanüle (10), bestimmt zum Ablegen eines Elements (26) in einem Körper eines Lebewesens, umfassend einen eine erste Handhabe (16) aufweisenden Kanülenhalter (14) und eine diesen zumindest bereichsweise umgebende und zu diesem axial verschiebbare, einen sich in der Kanüle erstreckenden Mandrin (24) aufweisende Hülse (18) mit zweiter Handhabe (20), wobei das Element durch Relativbewegung zwischen dem Mandrin und der das Element aufnehmenden Kanüle (10) in dem Körper ablegbar ist,
**dadurch gekennzeichnet**,
daß bei Druck auf die zweite von der Hülse (18) ausgehende Handhabe (20) die Hülse mit dem Kanülenhalter (14) durch zumindest ein von diesem ausgehendes Spreizelement (40) oder ein von dem Kanülenhalter oder der Hülse ausgehendes Rast- oder Hebelelement (54, 68, 80, 82) verrastbar ist.

2. Kanüle nach Anspruch 1,
**dadurch gekennzeichnet**,
daß der Kanülenhalter (14) aus einem unverrückbar die Kanüle (10) umgebenden Kanülenansatz (38) und einem diesen umgebenden und relativ zu diesem axial verschiebbaren die erste Handhabe (16) aufweisenden Hülsenkörper (48) besteht, daß von dem Kanülenansatz zumindest ein von der Kanülenspitze (12) weg in Richtung der Hülse (18) sich erstreckendes Spreizelement (40) ausgeht, welches sich bei Druck auf die zweite Handhabe gegen einen Abschnitt (46) der Hülse abstützt, und daß bei Bewegung der von dem Kanülenhalter (14) ausgehenden ersten Handhabe in Richtung der zweiten Handhabe der Hülsenkörper das Spreizelement in Ausgriff mit dem Abschnitt bringt und sodann den Ansatz mitzieht.

3. Kanüle nach Anspruch 1,
**dadurch gekennzeichnet**,
daß von der Hülse (18) ein verschwenkbares Rastelement wie Hebel (54) ausgeht, das gegen den Kanülenhalter (14) bzw. dessen Handhabe (16) abstützbar ist.

4. Kanüle nach Anspruch 3,
**dadurch gekennzeichnet**,
daß das Rastelement ein Winkelhebel (54) ist, dessen kurzer Schenkel (58) im Bereich der zweiten Handhabe, vorzugsweise entlang deren äußerer der Kanüle (10) abgewandten Fläche (60) verläuft.

5. Kanüle nach zumindest einem der vorhergehenden Ansprüche,
**dadurch gekennzeichnet**,
daß der Winkelhebel (56) in einer von der zweiten Handhabe (20) ausgehenden, Schwenkachse (56) für den Winkelhebel bildenden Aufnahme (57) festklemmbar ist.

6. Kanüle nach zumindest einem der vorhergehenden Ansprüche,
**dadurch gekennzeichnet**,
daß der kurze Schenkel (58) des Winkelhebels (54) in seinem freien Endbereich (62) an der zweiten Handhabe (20) abgestützt und/oder verrastbar ist und zwischen Abstützung bzw. Verrastung und der Schwenkachse (56) beabstandet zu der Außenfläche (60) der zweiten Handhabe (20) verläuft.

7. Kanüle nach zumindest Anspruch 1,
**dadurch gekennzeichnet**,
daß von dem Kanülenhalter (14) ein außenseitig entlang der Hülse (18) verlaufender und mit dieser verrastbares Hebelelement (68, 80, 82) ausgeht.

8. Kanüle nach zumindest Anspruch 7,
**dadurch gekennzeichnet**,
daß das Hebelelement (68) vorzugsweise über ein Scharnier wie Filmscharnier (76) mit der ersten Handhabe (16) verbunden ist und über eine radial oder seitlich zur Hülse (18) verlaufende Handhabe (78) erfaßbar und verschwenkbar ist.

9. Kanüle nach zumindest einem der vorhergehenden Ansprüche,
**dadurch gekennzeichnet**,
daß das Hebelelement (68) mit einem endseitigen Vorsprung (70) in der Hülse (18) arretierbar ist.

10. Kanüle nach zumindest einem der vorhergehenden Ansprüche,
**dadurch gekennzeichnet**,
daß bei der zu der Hülse (18) arretierten Kanülenhalterung (14) der endseitige Vorsprung (70) des Hebelelements (68) die Hülse (18) durchsetzt und in eine Aussparung (74) der Kanülenhalterung eingreift.

11. Kanüle nach zumindest einem der vorhergehenden Ansprüche,
**dadurch gekennzeichnet**,
daß das Hebelelement (68) in Richtung der Hülse (18) vorzugsweise werkstoffbedingt federvorgespannt ist.

12. Kanüle nach zumindest einem der vorhergehenden Ansprüche,
**dadurch gekennzeichnet**,
daß das Hebelelement (80, 82) bei fehlender Krafteinwirkung auf dieses beabstandet zur Hülse (18) verläuft.

13. Kanüle nach zumindest einem der vorhergehenden Ansprüche,
**dadurch gekennzeichnet**,
daß zwei Hebelelement (80, 82) von der ersten Handhabe (16) ausgehen.

14. Kanüle nach vorzugsweise einem der vorhergehenden Ansprüche,
**dadurch gekennzeichnet**,
daß das zumindest außenseitig nachgiebige Eigenschaften aufweisende Element (26) von einem Abschnitt (36) der Kanüle (10) festgeklemmt ist, in dem die Kanüle im Vergleich von zwischen dem Element und der Kanülenspitze (12) vorhandenen Querschnitt einen abweichenden Querschnitt oder eine Durchbrechung (28, 30) aufweist.

15. Kanüle nach Anspruch 14,
**dadurch gekennzeichnet**,
daß die Querschnitts- bzw. Lumenänderung durch eine zumindest bereichsweise umlaufende Sicke gebildet ist.

16. Kanüle nach Anspruch 14,
**dadurch gekennzeichnet**,
daß die Querschnitts- bzw. Lumenänderung durch zumindest eine Körnung (28, 30) eines Kanülenwandabschnitts gebildet ist.

17. Kanüle nach Anspruch 15,
**dadurch gekennzeichnet**,
daß die Querschnitts- bzw. Lumenänderung durch zumindest eine Durchbrechung (28, 30) in einem Kanülenwandabschnitt gebildet ist, wobei sich vorzugsweise der innere Rand (32, 34) der Durchbrechung in das Kanüleninnere erstreckt.

18. Kanüle nach Anspruch 16,
**dadurch gekennzeichnet**,
daß im Bereich des das Element (26) festklemmenden Abschnitts (36) der Kanüle eine Durchbrechung als Sichtfenster vorhanden ist.

19. Kanüle nach zumindest einem der vorhergehenden Ansprüche,
**dadurch gekennzeichnet**,
daß das Sichtfenster zumindest eine die Lumenänderung hervorrufende Durchbrechung (28, 30) ist.

## Claims

1. A cannula (10) for deposition of an object (26) in the body of a living organism, comprising a cannula holder (14) having a first handle (16) and a sleeve (18) with a second handle (20) surrounding said cannula holder (14) at least in part and being axially movable in relation thereto, and having a mandrin (24) extending inside said cannula, said object being depositable in the body by a relative movement between said mandrin and said cannula (10) receiving said object,
**characterized in that**
when pressure is applied to said second handle (20) extending from said sleeve (18) the latter can be engaged with said cannula holder (14) by at least one spreader element (40) extending from said cannula holder or by an engaging or lever element (54, 68, 80, 82) extending from said cannula holder or said sleeve.

2. A cannula according to Claim 1,
**characterized in that**
said cannula holder (14) comprises an immovable cannula shoulder (38) surrounding said cannula (10) and a sleeve body (48) surrounding said shoulder and axially movable relative thereto and incorporating said first handle (16), in that at least one spreader element (40) extending away from the cannula tip (12) and in the direction of said sleeve (18) extends from said cannula shoulder and rests against a section (46) of said sleeve when pressure is applied to said second handle, and that during movement of said first handle extending from said cannula holder (14) in the direction of said second handle said sleeve body disengages said spreader element from said section and moves said shoulder too.

3. A cannula according to Claim 1,
**characterized in that**
a swiveling engaging element such as a lever (54) extends from said sleeve (18) and can rest against said cannula holder (14) or its handle (16).

4. A cannula according to Claim 3,
**characterized in that**
said engaging element is an angled lever (54) whose short leg (58) is in the area of said second handle, preferably along its outer surface (60) facing away from said cannula (10).

5. A cannula according to at least one of the preceding claims,
**characterized in that**
said angled lever (56) can here be clampable in a receptacle (57) extending from said second handle (20) and forming the swivel axis (56) for said angled lever.

6. A cannula according to at least one of the preceding claims,
**characterized in that**
said short leg (58) of said angled lever (54) should rest on and/or engage with said second handle (20) at its free end part (62) and run between said rest/engaged connection and said swivel axis (56) at a distance from said outer surface (60) of said second handle (20).

7. A cannula according to Claim 1 at least,
**characterized in that**
a lever element (68, 80, 82) running on the outside along said sleeve (18) and engageable therewith extends from said cannula holder (14),

8. A cannula according to Claim 7 at least,
**characterized in that**
said lever element (68) is connected to said first handle (16) preferably by a hinge such as a film hinge (76) and can be gripped and swiveled by a handle (78) running radially or laterally to said sleeve (18).

9. A cannula according to at least one of the preceding claims,
**characterized in that**
said lever element (68) is lockable in said sleeve (18) by a projection (70) at its end.

10. A cannula according to at least one of the preceding claims,
**characterized in that**
when said cannula holder (14) is locked relative to said sleeve (18) said end projection (70) of said lever element (68) passes through said sleeve (18) and engages in a recess (74) of said cannula holder.

11. A cannula according to at least one of the preceding claims,
**characterized in that**
said lever element (68) is spring-pretensioned in the direction of said sleeve (18) preferably by virtue of the material.

12. A cannula according to at least one of the preceding claims,
**characterized in that**
said lever element (80, 82) is at a distance from said sleeve (18) when no force is exerted on the latter.

13. A cannula according to at least one of the preceding claims,
**characterized in that**
two lever elements (80, 82) extend from said first handle (16).

14. A cannula according to preferably one of the preceding claims,
**characterized in that**
said object (26) having yielding properties at least on the outside is clamped by a section (36) of said cannula (10) in which said cannula has a cross-section or aperture (28, 30) differing from that present between said object and said cannula tip (12).

15. A cannula according to Claim 14,
**characterized in that**
the cross-section or lumen reduction is achieved by a bead surrounding said cannula at least in parts.

16. A cannula according to Claim 14,
**characterized in that**
the cross-section or lumen reduction is achieved by at least one dimple (28, 30) of a cannula wall section.

17. A cannula according to Claim 15,
**characterized in that**
said cross-section or lumen reduction is achieved by at least one aperture (28, 30) in a cannula wall section, with the inner edge (32, 34) of said aperture preferably extending into the interior of said cannula.

18. A cannula according to Claim 16,
**characterized in that**
in the area of said cannula section (36) clamping said object (26) an aperture is provided as a vision panel.

19. A cannula according to at least one of the preceding claims,
**characterized in that**
said vision panel is at least one aperture (28, 30) causing the lumen change.

## Revendications

1. Canule (10), destinée au dépôt d'un élément (26) dans le corps d'un être vivant, comportant un support de canule (14) qui présente une première poignée (16), et comportant une douille (18) avec une deuxième poignée (20), laquelle douille entoure le support de canule au moins par parties et peut glisser axialement par rapport à ce dernier, la douille présentant un mandrin (24) s'étendant dans la canule, tandis que l'élément peut être déposé dans le corps par un déplacement relatif entre le mandrin et la canule (10) qui reçoit l'élément,
caractérisée en ce que par poussée sur la deuxième poignée (20) débordant de la douille (18), la douille peut être accrochée élastiquement avec le support de canule (14) par au moins un élément d'écartement (40) débordant de ce dernier ou par un élément d'accrochage élastique ou un élément de levier (54, 68, 80, 82) débordant du support de canule ou de la douille.

2. Canule selon la revendication 1, caractérisée en ce que le support de canule (14) est constitué d'un appendice de canule (38) qui entoure la canule (10) de manière immobile, et d'un corps de douille (48) qui présente la première poignée (16) et qui entoure l'appendice de canule et peut glisser axialement par rapport à ce dernier, en ce que de l'appendice de canule déborde au moins un élément d'écartement (40) qui s'étend à partir de la pointe (12) de la canule en direction de la douille (18) et qui, lors d'une poussée exercée sur la deuxième poignée, s'appuie contre une partie (46) de la douille, et en ce que lorsque la première poignée débordant du support de canule (14) est déplacée en direction de la deuxième poignée, le corps de douille décroche l'élément d'écartement de la partie et entraîne ainsi l'appendice.

3. Canule selon la revendication 1, caractérisée en ce que de la douille (18) déborde un élément pivotant d'accrochage élastique tel qu'un levier (54) qui peut s'appuyer contre le support de canule (14) ou sa poignée (16).

4. Canule selon la revendication 3, caractérisée en ce que l'élément d'accrochage élastique est un levier coudé (54) dont la courte branche (58) s'étend dans la région de la deuxième poignée, de préférence le long de la surface externe (60) de celle-ci qui n'est pas tournée vers la canule (10).

5. Canule selon au moins l'une des revendications précédentes, caractérisée en ce que le lever coudé (54) peut être immobilisé dans un logement (57) partant de la deuxième poignée (20) et formant axe de pivotement (56) pour le levier coudé.

6. Canule selon au moins l'une des revendications précédentes, caractérisée en ce que la courte branche (58) du levier coudé (54) s'appuie et/ou peut s'accrocher élastiquement sur la deuxième poignée (20) dans la région de son extrémité libre (62) et s'étend entre l'appui ou l'accrochage élastique et l'axe de pivotement (56), à distance de la surface externe (60) de la deuxième poignée (20).

7. Canule selon au moins la revendication 1, caractérisée en ce que du support de canule (14) part un élément de levier (68, 80, 82) qui s'étend le long de l'extérieur de la douille (18) et peut s'accrocher élastiquement à cette dernière.

8. Canule selon au moins la revendication 7, caractérisée en ce que l'élément de lever (68) est relié à la première poignée (16) de préférence par l'intermédiaire d'une charnière telle qu'une charnière en feuille (76), et peut être saisi et pivoté par l'intermédiaire d'une poignée (78) qui s'étend radialement ou latéralement par rapport à la douille (18).

9. Canule selon au moins l'une des revendications précédentes, caractérisée en ce que l'élément de levier (68) peut être bloqué dans la douille (18) au moyen d'une saillie d'extrémité (70).

10. Canule selon au moins l'une des revendications précédentes, caractérisée en ce que lorsque le support de canule (14) est verrouillé par rapport à la douille (18), la saillie d'extrémité (70) de l'élément de lever (68) traverse la douille (18) et s'engage dans une découpe (74) du support de canule.

11. Canule selon au moins l'une des revendications précédentes, caractérisée en ce que l'élément de levier (68) est précontraint élastiquement en direction de la douille (18), de préférence par les propriétés de son matériau.

12. Canule selon au moins l'une des revendications précédentes, caractérisée en ce que l'élément de lever (80, 82) s'étend à distance de la douille (18) lorsque qu'aucune force n'agit.

13. Canule selon au moins l'une des revendications précédentes, caractérisée en ce que deux éléments de levier (80, 82) partent de la première poignée (16).

14. Canule selon, de préférence, l'une des revendications précédentes, caractérisée en ce que l'élément (26), qui présente des propriétés de déformabilité au moins à l'extérieur, est immobilisé par une partie (36) de la canule (10), partie dans laquelle la canule présente soit une section transversale qui diffère de la section transversale entre l'élément et la pointe (12) de la canule, soit une perforation (28, 30).

15. Canule selon la revendication 14, caractérisée en ce que la modification de la section transversale ou du lumen est formée par un bourrelet au moins partiellement périphérique.

16. Canule selon la revendication 14, caractérisée en ce que la modification de la section transversale ou du lumen est formée par au moins une granularité (28, 30) d'une partie de la paroi de la canule.

17. Canule selon la revendication 15, caractérisée en ce que la modification de la section transversale ou du lumen est formée par au moins une perforation (28, 30) dans une partie de la paroi de la canule, le bord interne (32, 34) de la perforation s'étendant de préférence à l'intérieur de la canule.

18. Canule selon la revendication 16, caractérisée en ce que dans la région de la partie (36) de la canule qui immobilise l'élément (26), il existe une perforation qui sert de fenêtre d'observation.

19. Canule selon au moins l'une des revendications précédentes, caractérisée en ce que la fenêtre d'observation est au moins une perforation (28, 30) qui provoque la modification du lumen.
